(19)

**Europäisches Patentamt**

**European Patent Office**

**Office européen des brevets**

(11) **EP 4 119 192 A1**

(12) **EUROPEAN PATENT APPLICATION**

(43) Date of publication:
**18.01.2023 Bulletin 2023/03**

(51) International Patent Classification (IPC):
**A61N 5/10** (2006.01)    **G21K 5/04** (2006.01)
**H05H 7/00** (2006.01)    **H01J 3/12** (2006.01)

(21) Application number: **21185726.3**

(22) Date of filing: **15.07.2021**

(52) Cooperative Patent Classification (CPC):
**A61N 5/1075; G21K 5/04; H01J 3/12; H05H 7/001;**
A61N 2005/1087; H05H 2007/005; H05H 2277/11

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Paul Scherrer Institut**
**5232 Villigen PSI (CH)**

(72) Inventors:
• **MARADIA, Vivek**
  **8142 Uitikon Waldegg (CH)**

• **PSOROULAS, Serena**
  **8050 Zürich (CH)**
• **SCHIPPERS, Jacobus Maarten**
  **5236 Remigen (CH)**
• **MEER, David**
  **5200 Brugg (CH)**

(74) Representative: **Fischer, Michael**
**Siemens AG**
**Postfach 22 16 34**
**80506 München (DE)**

(54) **OPTIMIZED MATCHING OF BEAM EMITTANCE AND COLLIMATION SYSTEM TO MAXIMIZE TRANSMISSION THROUGH BEAMLINE**

(57)    The present invention provides a method to design a beam collimation system for cyclotron-based particle therapy beamline. This method provides the specifications of two asymmetric collimators, which allow transporting larger emittance through beamline while having symmetric emittance in both planes but with different beam sizes and different divergences in both planes. The first asymmetric collimator selects an asymmetric beam size and the second collimator selects an asymmetric beam divergence in both planes. The aperture of the second collimator depends on the aperture of the first collimator. This arrangement allows transporting larger emittance through beamline for lower energy, with the goal of achieving higher beam current transmission compared to the prior art. Preferably, this collimator system is integrated after the energy degrader but it is possible to use a similar collimation system at any location along the beamline. These collimators can be multileaf or fixed aperture collimators.

FIG. 1

EP 4 119 192 A1

**Description**

The present invention relates to a cyclotron based charged particle therapy apparatus used for proton or ion beam radiation therapy. More particularly, this invention relates to a beam collimation system to achieve symmetric emittance in both planes to obtain maximum possible beam current transmission through beamline.

The use of radiation therapy for cancer cure is about finding a balance between the risks and benefits of radiation on healthy organs and cancer tumors. Proton therapy allows sparing healthy organs better than standard radiation therapy. However, the delivery of proton therapy is prone to uncertainties caused by anatomical changes and motion during the treatment and between the treatment fractions, which may compromise its effectiveness.

This limits the use of proton therapy technology to cure moving tumors. Such treatments will necessarily require some means for mitigating the interplay effect between the motions in the patient and the beam delivery. The use of high-intensity proton beams to treat patients with high dose rates would open the possibility of treating moving tumors. With high-intensity beams, it is possible to use motion mitigation techniques for moving targets, FLASH proton therapy and it is possible to reduce the total treatment time. Additionally, short treatment time can increase patient throughput. This will help with reducing the proton therapy treatment cost.

A typical proton therapy system can be divided into three different parts. The first part is an accelerator, which produces energetic charged particles. The second part is the fixed beamline, which transports the beam from an accelerator exit to the rotating beamline. The third part is the gantry (rotating beamline), which delivers the beam to the target in different directions. Most of the proton therapy facilities use a cyclotron to produce energetic protons.

One issue with cyclotron-based facilities is the need to lower the beam energy from the fixed value provided by the accelerator to the one needed for the treatment. Usually, this is obtained by letting the beam pass through a so-called energy selection system (ESS). First, the beam passes through a certain thickness of a low atomic number material (so-called 'degrader'), losing energy until the desired energy is reached. This causes additional scattering and increases the energy spread of the beam. For this reason, a degrader system is always followed by a phase space selection system and ESS, which selects the emittance of the beam and the right energy, lowering the momentum spread. Such an energy modulation system has the drawback of introducing an energy-dependent beam current transmission due to the beam losses at the ESS. The design of ESS and these losses are described in van Goethem et al., 2009.

At PSI, for the lowest energies, the transmission through the energy selection system (which follows a graphite degrader) is less than 1%. In proton therapy, these low transmissions for lower energies cause an undesirable increase in treatment time, which complicates the treatment of moving tumors.

Two main approaches can increase beam current transmission for lower energies. The first method is reducing the emittance growth by choosing a different degrader material. New degrader materials (e.g. $B_4C$) can increase the transmission by the order of 10-20%. The second option to improve the transmission is optimizing beam characteristics (emittance) to reduce the losses and transport a higher number of particles along the beamline. A higher emittance (factor 3 or higher) will result in a proportionally higher transmission at low energies. If it is possible to transport higher (factor 3 or more) emittance, it will increase the low energy transmission by factors. This will open the possibilities to achieve very high-intensity proton beams.

The beam intensity and beam divergence profiles can be described by normal distributions, characterized by their standard deviations (sigma). In a specific transversal plane (X or Y plane) emittance is defined from the two-sigma beam size and beam divergence estimates. At the location of the beam waist, emittance is defined as the multiplication of two-sigma beam size (in mm) and two-sigma beam divergence (in mrad). The factor pi is written separately but it is not included in the numerical value.

$$\varepsilon_x = x * x' \quad \texttt{(pi*mm*mrad)}$$

$$\varepsilon_y = y * y' \quad \texttt{(pi*mm*mrad)}$$

where

$\varepsilon_x$ = two sigma emittances in X-plane at beam waist,
$\varepsilon_y$ = two sigma emittances in Y-plane at beam waist,
$x$ = two sigma beam size in X-plane (mm),
$y$ = two sigma beam size in Y-plane (mm),
$x'$= two sigma beam divergence in X-plane (mrad) and,
$y'$ = two sigma beam divergence in Y-plane (mrad)

Symmetric emittance:
When the emittance of the beam is the same in both planes, it is called symmetric emittance. The shape of the emittance ellipse may be different in a different plane, but the area is the same.
The beam with symmetric emittance needs not necessarily be round.
Asymmetric emittance:
When the emittance of the beam is different in both planes, called asymmetric emittance. The shape of the emittance ellipse and the area are always different in a different plane. The beam with asymmetric emittance can be round with different divergence in both planes.
Beam characteristics requirements at the entrance of the entrance of the gantry
The beam-transport system of the beamline (from energy degrader to gantry entrance) is characterized by its transfer matrix $R_{gain}$.

$$R_{gain} = \begin{bmatrix} R_{11} & R_{12} & R_{13} & R_{14} & R_{15} & R_{16} \\ R_{21} & R_{22} & R_{23} & R_{24} & R_{25} & R_{26} \\ R_{31} & R_{32} & R_{33} & R_{34} & R_{35} & R_{36} \\ R_{41} & R_{42} & R_{43} & R_{44} & R_{45} & R_{46} \\ R_{51} & R_{52} & R_{53} & R_{54} & R_{55} & R_{56} \\ R_{61} & R_{62} & R_{63} & R_{64} & R_{65} & R_{66} \end{bmatrix}$$

$R_{11}$, $R_{12}$, $R_{21}$, and $R_{22}$ terms describe the particle trajectory in the horizontal plane. $R_{11}$ = Gain in beam size in X-plane, $R_{12}$ and $R_{21}$ give the correlation between beam size and beam divergence and, $R_{22}$ Gain in beam divergence. Similar way, $R_{33}$, $R_{34}$, $R_{43}$, and $R_{44}$ terms describe the particle trajectory in the vertical plane. $R_{13}$, $R_{14}$, $R_{23}$, $R_{24}$, $R_{31}$, $R_{32}$, $R_{41}$, and $R_{42}$ terms describe the coupling between horizontal and vertical plane. If they all are zero, there is no coupling. The $R_{16}$, $R_{26}$, $R_{36}$, and $R_{46}$ terms describe the dispersion function and its derivatives in the horizontal and vertical plane, respectively. The term $R_{61}$, to $R_{65}$ are zero and $R_{66}$ = 1, as the momentum is not changed along the beamline.
In the description, a standard proton therapy facility layout with a rotating gantry is assumed. This layout translates into a specific requirement for the beam emittance. The method however can be easily generalized to other beam emittance constraints (e.g. for a fixed beamline).
Transporting the beam through the rotating gantry becomes complicated if the beam enters the gantry with different beam sizes, or with different divergence in the horizontal and vertical plane, (Asymmetric beams). To achieve gantry-angle independent beam transport, it is important to have the same beam size and same divergence in both planes (same emittance in both planes). It is also important to have a beam waist (no correlation between beam size and divergence) at the entrance of the gantry.
In summary, it is required to achieve the following beam parameters at the gantry entrance to achieve gantry angle independent beam transport.

- Beam waist at the entrance of the gantry ($R_{12} = R_{21} = R_{34} = R_{43} = 0$)
- Same beam size and same divergence (same emittance) in both plane at the entrance of the gantry ($\varepsilon_x = \varepsilon_y$ and $R_{11} = R_{33}$ and $R_{22} = R_{44}$)

To get the same beam size and same divergence at the gantry entrance without creating losses at the coupling point, it is required to transport the same emittance along the beamline in both planes from the degrader to the gantry entrance. To transport the same emittance in both planes, it is needed to have symmetric emittance in both planes from the collimator system.

**Problem in current state-of-the-art facilities**

As mentioned above, cyclotron-based facilities need an energy degrader and an ESS. The emittance increase after degrader for lower energies is beyond the beam transport system vacuum pipe aperture. To prevent losses along the beamline, it is unavoidable to use an emittance selection collimator to limit the emittance such that it fits the beam transport system.
As an example, one will use the ESS beamline of the PROScan facility at the Paul Scherrer Institute (PSI), located at CH-5232 Villigen PSI, Switzerland. The system is illustrated in Fig. 1. At the exit of cyclotron 11, a quadrupole triplet 12 focuses the 250 MeV beam at the center of the degrader 13. After the degrader, a first round/symmetric collimator 14 selects the beam size, and the second round/symmetric collimator 15 selects the beam divergence. Then the beam passes the following energy selection system, consisting of two 58° bending magnets 18, several quadrupoles, and slits 20 for the momentum selection. After the ESS, the beam is transported to the entrance of the different treatment rooms (gantries).

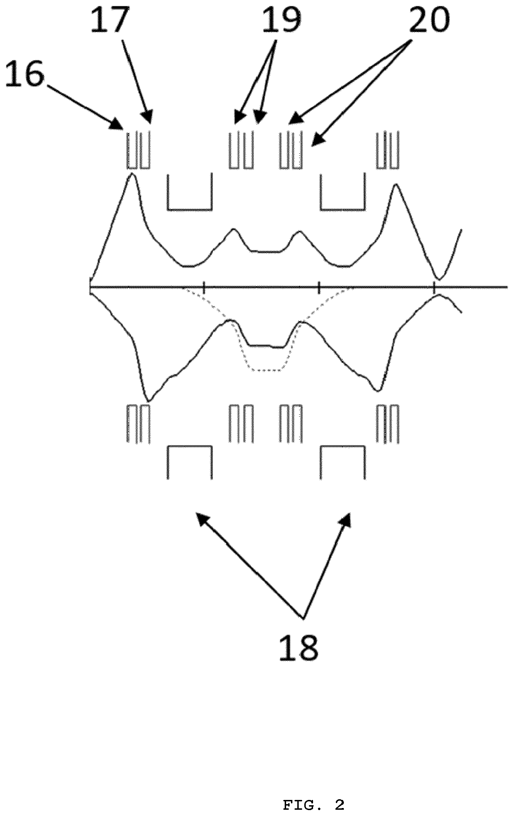

To match the emittance after the degrader to the following beamline in the PROScan system, the aperture of the collimator 14 selects a small beam size in both planes. The collimator 15 selects the beam divergence to get the same emittance in both planes. As quadrupole 16 focuses the beam in Y-plane, it is possible to choose a large divergence in Y-plane while it is defocusing the beam in the X-plane, which limits the selection of the divergence in the X-plane. The quadrupole 16 limits the amount of emittance, which can be transported through a beamline. In Fig. 2 an example envelope is shown; with 3 mm beam size selected in collimator 14, the divergence acceptance in Y-plane is 27 mrad and in X-plane, it is 11 mrad. With these particular collimator settings, emittance acceptance in Y-plane is 81 pi*mm*mrad and in X-plane is 33 pi*mm*mrad.

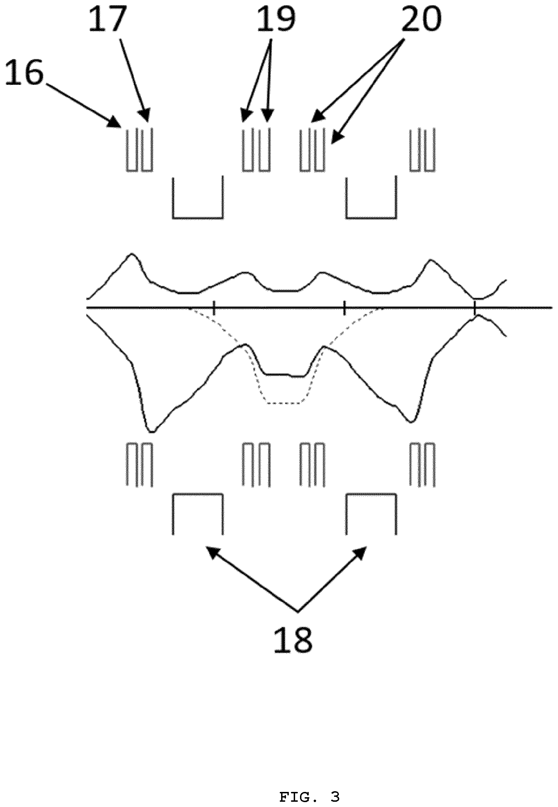

However, to get a gantry angle independent beam size at the ISO center, it is mandatory to transport the same emittance in both planes. Fig. 3 illustrates the beam envelope starting from collimator 14 that fulfills the symmetric emittance requirement. It can be easily seen, that the transported emittance in Y is strongly reduced with respect to Fig. 2. Reducing the emittance with collimator 14 and 15 at low energy causes losses of more than 99% in the PROScan facility.

One of the options to increase the emittance transported through the beamline is to increase the aperture of the collimator 14 and select the larger beam size instead of 3 mm. However, it is not possible to select a very large beam size in Y-plane because of the small aperture size of the first bending magnet of the ESS in the Y-plane. A large beam size in Y-plane will lead to beam losses in the bending magnet in Y-plane. Therefore, there is a limitation of beam size in the Y-plane.

Another possibility is not to use emittance selection collimators 14 and 15 and use the quadrupole 16 immediately after the degrader. However, this solution would also cause significant beam losses in the following magnets apertures. The main goal of the beam optics design is to avoid losses in any components along the beam transport system other than collimators.

Therefore, it is the objective of the present invention to provide a beam shaping systems that includes beam selection collimators that are still advisable, but their specifications need to be optimized to maximize transmission.

This objective is achieved according to the present invention by a particle beam transport system for the delivery of particle beam therapy wherein the particle beam has substantially the same emittance in both planes perpendicular to the direction of propagation of the particle beam, comprising:

a) a particle beam source, such as an ion- or proton accelerator,
b) a beam transport line,
c) a rotatable gantry, wherein:
d) the particle beam is transported by the beam transport line from the particle beam source to the rotatable gantry;
e) the beam transport line comprises a degrader and a collimator system disposed downstream of the degrader; and
f) the collimator system comprises a set of at least a first asymmetric collimator and a second asymmetric collimator to achieve the same emittance of the particle beam in both planes perpendicular to the propagation direction of the particle beam.

Therefore, the present invention proposes a design of the collimation system that can open the possibility to transport higher emittance through the particle beamline while achieving the same emittance in both plane. Additionally, the new device is designed in a way that it can easily replace the prior art device without bigger modifications along the beamline. With the help of the present invention, it is possible to increase the transmission by factors for lower energies.

In the prior art, the beamline of cyclotron based particle therapy apparatus uses a round/symmetric beam size selection collimator, to limit the emittance transported through the beamline. For this reason, a very small part of the total emittance for lower energy beams is transported along the beamline, causing low beam current transmissions (<1%). The present invention provides the design of the collimation system through which it is possible to transport higher emittance through beamline while having the same emittance in both planes.

In a preferred embodiment of the present invention, the collimation system may comprise a set of two asymmetric collimators wherein the first collimator selects the size of the beam and the second collimator selects the divergence of the beam. The design of the collimation system is mainly divided into three parts:

(1) Calculate the beam size and beam divergence values required in both planes through the collimation system to satisfy beam emittance constraints in both planes,
(2) Design of the beam size selection collimator aperture, and
(3) Design of the beam divergence selection collimator aperture.

A further preferred embodiment of the present invention is a set of two asymmetric collimators, which selects different beam sizes and different beam divergence in both planes in a way, that the same emittance in both planes is achieved after the collimation system. By using this design of the collimation system, it is possible to transport higher emittance (factor 3 or more) through the beamline. As a result, it is possible to transport more particles through the beamline for lower energies thereby increasing the transmission by factors.

Another preferred embodiment of the present invention can be achieved when the first asymmetric collimator selects an asymmetric beam size and the second asymmetric collimator selects the asymmetric beam divergence and wherein the particle beam has as seen downstream of the collimator system substantially the same emittance in both plane, but different beam size and different divergence in both plane.

Further, upstream of the first asymmetric collimator and between the two asymmetric collimators, the beam path can be free of beam focusing means. This measure is advantageous in the trimming of the particle beam in terms of its emittance.

Advantageously, the first and/or the second asymmetric collimator can be realized as multi-leaf collimator and/or as a set of switchable collimators at fixed aperture.

Preferred embodiments of the present invention are described in more detail with reference to the attached drawings which depicts in:

Fig. 1    schematically a configuration of the energy selection system (ESS) of a medical particle beam facility;

Fig. 2    the beam optics calculation with an emittance of 81 pi*mm*mrad in Y-plane (Beam size: 3 mm, Beam divergence: 27 mrad) and 33 pi*mm*mrad in X-plane (Beam size: 3 mm, Beam divergence: 11 mrad)

Fig. 3    the beam optics calculation with an emittance of 33 pi*mm*mrad in both plane (Beam size: 3 mm, Beam divergence: 11 mrad)

Fig. 4    the steps of a method for designing a collimation device;

Fig. 5    the steps of a method for designing a beam size selection collimator;

Fig. 6    the beam optics calculation with emittance of 60 pi*mm*mrad in both plane (x = 5.3 mm, x'= 11 mrad, y = 2.2 mm, y' = 27 mrad); and

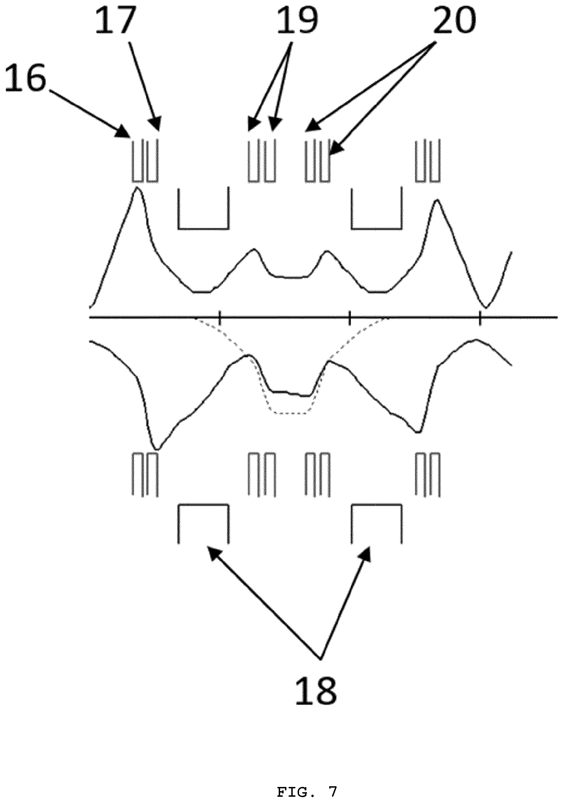

Fig. 7    the beam optics calculation with emittance of 100 pi*mm*mrad in both plane (x = 9.1 mm, x'= 11 mrad, y = 3.7 mm, y' = 27 mrad).

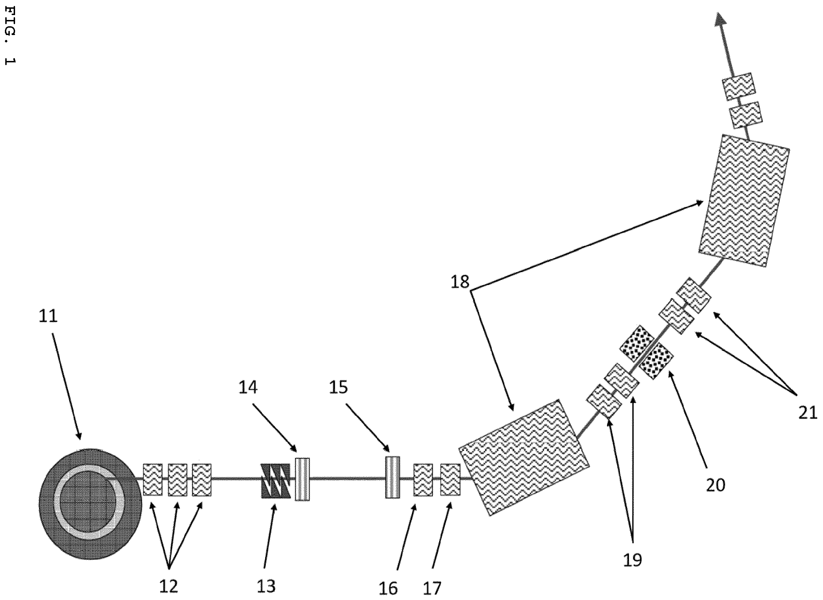

In a conventional cyclotron based particle therapy beamline, as illustrated in Fig. 1, a round collimator 14 is used to get the same beam size in both plane and a round collimator 15 is used to select the same beam divergence in both plane, eventually to obtain the same emittance in both planes to achieve gantry angle independent beam size at ISO center.

To design the beam optics for the ESS, it is required to fulfill the following three conditions at the emittance selection collimators:

(1) Same emittance in both planes after the collimator 15.
(2) Limitation of maximum beam size in Y-plane due to the limited aperture size of the first bending magnet of the ESS in Y-plane
(3) Limitation of maximum divergence in the X-plane due to the quadrupole function of focusing the beam in one plane and defocus the beam in another direction.

In addition to fulfilling these three conditions, it is important to find the best solution to transport higher emittance through the beamline.

The present invention provides a practical solution to transport higher emittance through the beamline by using two asymmetric collimators. It is possible to choose the beam size selection collimator 14 aperture in a way that one gets a large beam size in X-plane and small beam size in Y-plane. In addition, it is possible to choose beam divergence selection collimator 15 aperture in a way that allows small divergence in X-plane and large divergence in Y-plane while keeping the same emittance in both planes after the emittance selection collimators. In this way, one is able to increase the emittance transported through the beamline in both planes while having the same emittance in both planes but with different beam sizes and different divergence in both planes. This method of designing a collimation system is independent of which of the two coordinates (X or Y) is the dispersive plane.

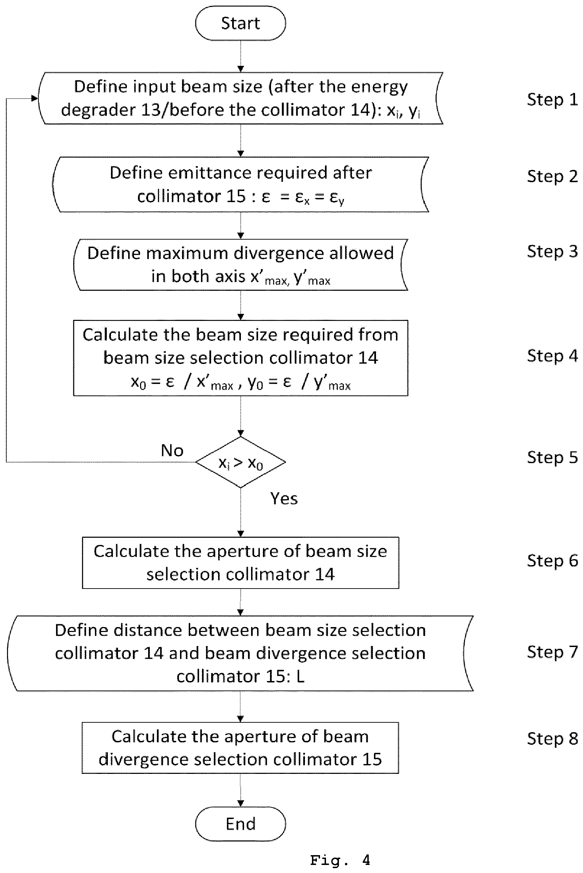

Fig. 4 illustrates a flowchart of the method for designing a collimation device. This collimation device is a combination of two asymmetric collimators placed one to two meters apart of each other. The function of the first collimator 14 is to select the size of the beam; it is placed after the energy degrader 13. The function of the second collimator 15 is to select the beam divergence. To make the design method of the collimation device easy to understand, the method is described using two example cases: an emittance goal of 60 pi*mm*mrad and 100 pi*mm*mrad respectively. Such emittances are a few factors larger than commonly used in proton therapy facilities.

The method of the collimation device design comprises the following steps:

**Step 1:** Define the beam parameters after a degrader 13. Generally, from a cyclotron 11, one gets a very narrow beam. After that, one uses a quadrupole triplet 12 to focus the beam at the center of the degrader 13 and tries to achieve a small beam size after the degrader 13 for lower energies. The degrader 13 will enlarge the beam size, while the triplet 12 can be used to focus the incoming beam and thus modify the output beam size according to the requirements. One such requirement is that for example, the beam size is constant for all energies after the degrader 13. By studying the effect of beam focusing and degrader spreading, the beam parameters after the degrader 13 can be easily defined. In this example, one defines the beam size of $x_i = y_i$ = 12 mm in both plane after the degrader 13.

**Step 2:** Define the emittance required after the collimator 15. The emittance is usually constrained by the following beamline, for example, the requirement of beam parameters at the entrance of the gantry. To achieve gantry angle independent beam optics, it is required to transport the same emittance in both planes. The aim is to transport factor 2-3 higher emittance through the ESS to achieve higher transmission. For example cases, one defines the emittance of 60 pi*mm*mrad and 100 pi*mm*mrad.

**Step 3:** Define the maximum divergence allowed in both planes. In the present example, after the degrader 13, the beam is diverging in both planes and a quadrupole magnet 16 is used to focus the beam in the Y-plane. The quadrupole 16 focuses the beam in one plane and defocuses the beam in another plane. As quadrupole 16 focuses the beam in Y-plane, it will defocus the beam in X-plane. This will lead to different divergence acceptance in both planes. To find out the maximum divergence allowed in both planes, it is required to do beam optics calculation using matrix multiplication. Matrix multiplication can provide such an estimate. For a fixed beam size above, different envelopes will be generated with increasing beam divergences, and the iteration stops as soon as the envelope reaches the aperture of the beamline. The maximum divergence found is the initial guess for the definition of the divergence collimator. For the present example cases, one can choose the beam divergence collimator aperture in a way that we can get $x'_{max}$ = 11 mrad in X-plane and $y'_{max}$ = 27 mrad in Y-plane.

**Step 4:** In this step, the beam size required from beam size selection collimator 14 is calculated. From step 2, one got the emittance value of 60 pi*mm*mrad and 100 pi*mm*mrad. Case 1 is to get emittance of 60 pi*mm*mrad and case 2 is to get emittance of 100 pi*mm*mrad in both planes.

- **Case 1:** 60 pi*mm*mrad

  Beam size required in X-plane = $x_0 = \varepsilon / x'_{max}$ = 5.5 mm
  Beam size required in Y-plane = $y_0 = \varepsilon / y'_{max}$ = 2.2 mm

- **Case 2:** 100 pi*mm*mrad

  Beam size required in X-plane = $x_0 = \varepsilon / x'_{max}$ = 9.1 mm
  Beam size required in Y-plane = $y_0 = \varepsilon / y'_{max}$ = 3.7 mm

**Step 5:** It is important to check that the beam size required from the beam size selection collimator 14 ($x_0$) is smaller than the beam size after the degrader 13 ($x_i$). For the present example cases, the beam sizes follow the following relation: $x_i < x_0$. If the above relation is not satisfied, it is possible to increase the beam size after the degrader 13 by defocusing the beam in X-plane using the quadrupole triplet 12 and achieve the above relation. The increase should however (if possible) limit the losses in the collimator.

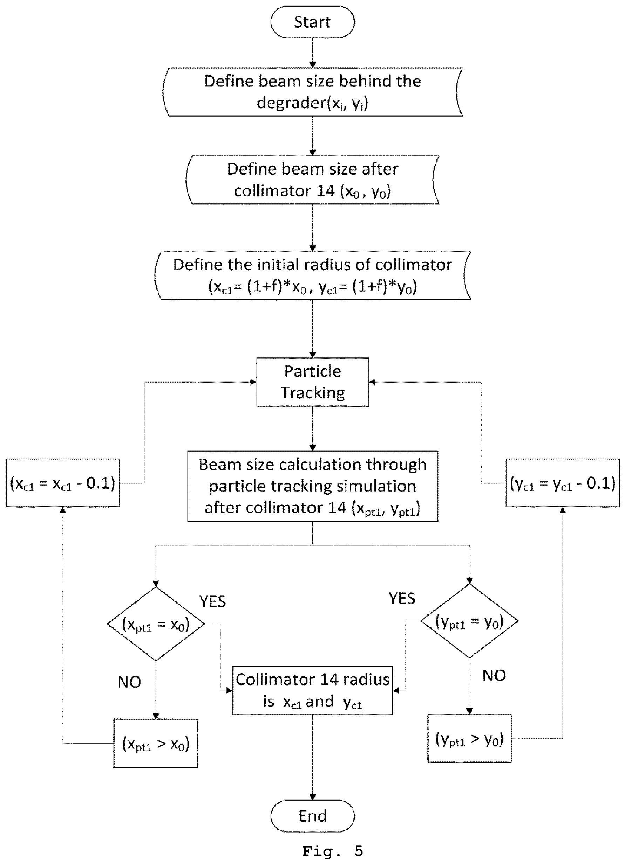

**Step 6:** Fig. 5 illustrates a flowchart of a method for designing the beam size selection collimator 14. It is assume that the beam after the degrader 13 follows a Gaussian distribution. However, when the collimator cuts the beam, the beam after the collimator is not a gaussian beam. Therefore, it is important to find out the equivalent Gaussian parameter of the collimated beam for consistency with the emittance definition and the following transport. For this purpose, the use of particle tracking simulation is suggested to find out the beam size after the collimation using the inputs from step 4 (the beam size required from beam size selection collimator 14 ($x_0$, $y_0$)) and step 1 (the beam size after the degrader $(x_i, y_i)$). An initial guess for the collimator size can be put at $x_{c1} = (1+f)^*x_0$ and $y_{c1} = (1+f)^*y_0$; it is recommended to choose f > 0.3, 0.4 since the collimator size will be larger than $x_0$ and $y_0$. With these input beam size parameters and the initial radius of the collimator, a particle tracking simulation is performed and the beam size after the collimator 14 $(x_{pt1}, y_{pt1})$ is calculated. As the initial value of collimator radius is factor 1+f larger, the following result are achieved:

$X_{pt1} > x_0$ and $y_{pt1} > y_0$

Now, one will start reducing the $x_{c1}$ and $y_{c1}$ value by 0.1 until one gets the following relation between simulated beam size and required beam size after collimator 14.

$x_{pt1} = x_0$ and $y_{pt1} = y_0$

- **Case 1:** 60 pi*mm*mrad ($x_0$ = 5.5 mm, $y_0$ = 2.2 mm and, $x_i = y_i$ = 12 mm)
  By doing particle tracking simulation, one gets a collimator 14 radius of $x_{c1}$ = 5.8 mm and $y_{c1}$ = 2.2 mm
- **Case 2:** 100 pi*mm*mrad ($x_0$ = 9.1 mm, $y_0$ = 3.7 mm and, $x_i = y_i$ = 12 mm)
  By doing particle tracking simulation, one gets a collimator 14 radius of $x_{c1}$ = 11.2 mm and $y_{c1}$ = 3.6 mm.

**Step 7:** In this step, it is required to define the distance L between beam size selection collimator 14 and beam divergence selection collimator 15. In the present example, L = 1 m.

**Step 8:** The last step of the collimator design is to calculate the aperture of the beam divergence collimator 15. One knows the maximum divergence value from step 3, and the distance between collimator 14 and collimator 15 from step 7. The radius of the collimator 15 $(x_{c2}, y_{c2})$ is thus:

$$\tan x'_{max} = \frac{x_{c2}}{L}$$

$$\therefore x_{c2} = \tan x'_{max} * L$$

Similar way,

$$\therefore y_{c2} = \tan y'_{max} * L$$

- **Case 1:** 60 pi*mm*mrad
  Collimator 15 radius: $x_{c2}$ = 13.6 mm and $y_{c2}$ = 33.5 mm
- **Case 2:** 100 pi*mm*mrad
  Collimator 15 radius: $x_{c2}$ = 13.6 mm and $y_{c2}$ = 33.5 mm

In summary, to transport a beam emittance of 60 pi*mm*mrad and of 100 pi*mm*mrad through the energy selections system ESS, following aperture radius of collimator 14 and collimator 15 are needed:

**Table 1: Required aperture radius of collimator 14 and 15 to achieve 60 pi*mm*mrad and 100 pi*mm*mrad emittance in both planes**

| Emittance (pi*mm*mrad) | Collimator 14 radius in X-plane (mm) | Collimator 14 radius in Y-plane (mm) | Collimator 15 radius in X-plane (mm) | Collimator 15 radius in Y-plane (mm) |
|---|---|---|---|---|
| 60 | 5.8 | 2.2 | 13.6 | 33.5 |
| 100 | 11.2 | 3.6 | 13.6 | 33.5 |

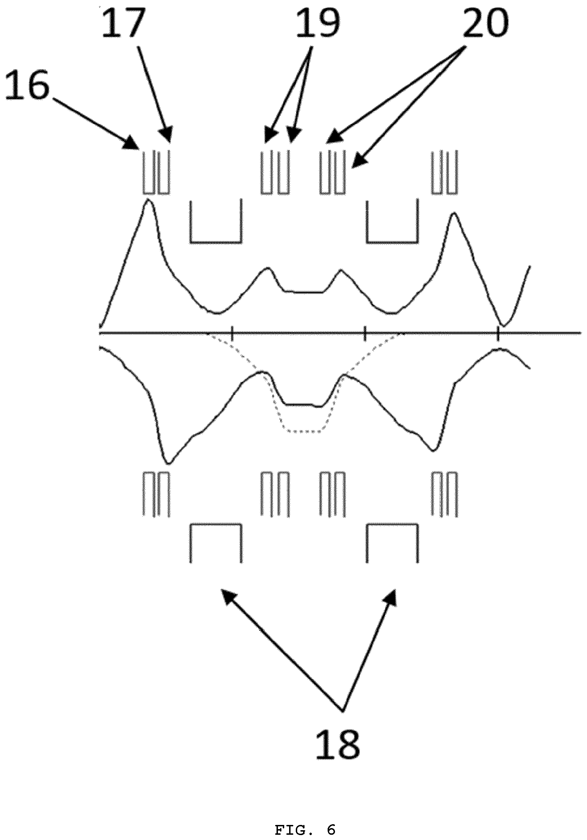

Fig. 5 illustrates the beam envelope of transporting a beam with an emittance of 60 pi*mm*mrad through the ESS by using a combination of the asymmetric collimators 14, 15. In a similar way, Fig. 6 illustrates the beam envelope of transporting a beam with an emittance of 100 pi*mm*mrad through the ESS by using a combination of the asymmetric collimators 14, 15.

The use of the combination of two asymmetric collimators 14, 15 - in general an asymmetric collimator combination comprising at least two asymmetric collimators - is very important to increase the transmission for lower and middle energy ranges, where the emittance after the degrader 13 is very large. For higher energies, the emittance growth after the degrader 13 is not significantly large; therefore, for higher energy, it is possible to use the symmetric collimators or asymmetric collimators with smaller aperture size.

To achieve a different aperture size for the collimator system, these are two possibilities suggested here. The first option is to use the movable stack of few different aperture collimators. For middle and lower energy, it is possible to use a large aperture asymmetric collimator and for higher energy, it is possible to move the collimator stack to a different collimator position with a small aperture asymmetric collimator or small aperture round/symmetric collimator. The second possible option is to use a multileaf collimator. By moving the leafs of the collimator, it is possible to achieve an asymmetric collimator as well as a round/symmetric collimator setting. With this option, by using a different combination of apertures, it is possible to select a wide range of emittance from emittance selection collimators.

The foregoing has outlined rather broadly the features and technical advantages of the present invention so that the detailed description of the invention that follows may be better understood. Additional features and advantages of the invention will be described hereinafter, which form the subject of the claims of the invention. It is evident that a person skilled in the art may conceive several equivalent embodiments or other ways of expressing the present invention.

**Claims**

1. A particle beam transport system for the delivery of particle beam therapy wherein the particle beam has substantially the same emittance in both planes perpendicular to the direction of the particle beam, comprising:

   a) a particle beam source (11), such as an ion- or proton accelerator,
   b) a beam transport line,
   c) a rotatable gantry, wherein:
   d) the particle beam is transported by the beam transport line from the particle beam source to the rotatable gantry;
   e) the beam transport line comprises degrader (13) and a collimator system disposed downstream of the degrader (13); and
   f) the collimator system comprises a set of at least a first asymmetric collimator (14) and a second asymmentric collimator (15) to achieve the same emittance of the particle beam in both planes perpendicular to the propagation direction of the particle beam.

2. The particle beam transport system according to claim 1, wherein the first asymmetric collimator (14) selects an asymmetric beam size and the second asymmetric collimator (15) selects the asymmetric beam divergence and wherein the particle beam has as seen downstream of the collimator system substantially the same emittance in both plane, but different beam size and different divergence in both plane.

3. The particle beam transport system according to claim 1 or 2 wherein upstream of the first asymmetric collimators (14) and between the two asymmetric collimators (14, 15), the beam path is free of beam focusing means.

4. The particle beam transport system according to any of the preceding claims, wherein the first and/or the second asymmetric collimator (14, 15) are realized as multi-leaf collimator and/or as a set of switchable collimators at fixed aperture.

FIG. 1

FIG. 2

FIG. 3

Fig. 4

Fig. 5

FIG. 6

FIG. 7

Europäisches
Patentamt

European
Patent Office

Office européen
des brevets

# EUROPEAN SEARCH REPORT

Application Number

EP 21 18 5726

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 107 648 746 A (CRYOELECTRA GMBH) 2 February 2018 (2018-02-02) * paragraphs [0001], [0094] – [0103]; figures 1–3 * | 1–4 | INV. A61N5/10 G21K5/04 H05H7/00 H01J3/12 |
| A | EP 2 308 561 A1 (ION BEAM APPLIC [BE]) 13 April 2011 (2011-04-13) * paragraph [0032] * | 1–4 | |
| A | CN 106 211 535 B (CHINA INST ATOMIC ENERGY) 24 August 2018 (2018-08-24) * figures 1–4 * | 1–4 | |
| A | US 10 998 158 B1 (LEWELLEN JOHN [US] ET AL) 4 May 2021 (2021-05-04) * claims 1–3; figure 5A * | 1–4 | |

| TECHNICAL FIELDS SEARCHED (IPC) |
|---|
| A61N H05G G21K H05H H01J |

The present search report has been drawn up for all claims

| Place of search | Date of completion of the search | Examiner |
|---|---|---|
| Munich | 23 December 2021 | Kajzar, Anna |

EPO FORM 1503 03.82 (P04C01)

## ANNEX TO THE EUROPEAN SEARCH REPORT
## ON EUROPEAN PATENT APPLICATION NO.

EP 21 18 5726

This annex lists the patent family members relating to the patent documents cited in the above-mentioned European search report.
The members are as contained in the European Patent Office EDP file on
The European Patent Office is in no way liable for these particulars which are merely given for the purpose of information.

23-12-2021

| Patent document cited in search report | | Publication date | Patent family member(s) | | Publication date |
|---|---|---|---|---|---|
| CN 107648746 | A | 02-02-2018 | NONE | | |
| EP 2308561 | A1 | 13-04-2011 | AT | 512696 T | 15-07-2011 |
| | | | BR 112012006799 A2 | | 24-05-2016 |
| | | | CN | 102596316 A | 18-07-2012 |
| | | | DK | 2308561 T3 | 03-10-2011 |
| | | | EP | 2308561 A1 | 13-04-2011 |
| | | | EP | 2482928 A1 | 08-08-2012 |
| | | | ES | 2368113 T3 | 14-11-2011 |
| | | | JP | 5096644 B1 | 12-12-2012 |
| | | | JP | 2013505757 A | 21-02-2013 |
| | | | KR | 20120107939 A | 04-10-2012 |
| | | | US | 2013001432 A1 | 03-01-2013 |
| | | | US | 2013187060 A1 | 25-07-2013 |
| | | | WO | 2011036254 A1 | 31-03-2011 |
| CN 106211535 | B | 24-08-2018 | NONE | | |
| US 10998158 | B1 | 04-05-2021 | NONE | | |

EPO FORM P0459

For more details about this annex : see Official Journal of the European Patent Office, No. 12/82